Europäisches Patentamt

(19) European Patent Office

Office européen des brevets

(11) Publication number : **0 521 685 A2**

(12) **EUROPEAN PATENT APPLICATION**

(21) Application number : **92305996.8**

(22) Date of filing : **29.06.92**

(51) Int. Cl.$^5$ : **B02C 19/06, B02C 23/10**

(30) Priority : **05.07.91 GB 9114589**

(43) Date of publication of application :
**07.01.93 Bulletin 93/01**

(84) Designated Contracting States :
**AT BE CH DE DK ES FR GR IT LI LU MC NL PT SE**

(71) Applicant : **ANGLIAN WATER COMMERCIAL DEVELOPMENTS LTD.**
**Anglian House, Ambury Road**
**Huntingdon, Cambridgeshire PE18 6NZ (GB)**

(72) Inventor : **Durrant, Helen Elizabeth**
**58 North End, Meldreth**
**Royston, Herts (GB)**
Inventor : **Pugh, Shirley Yvonne Ruth**
**15 Tall Trees**
**Royston, Herts (GB)**

(74) Representative : **Nash, Keith Wilfrid**
**KEITH W. NASH & Co. Pearl Assurance House**
**90-92 Regent Street**
**Cambridge CB2 1DP (GB)**

(54) **Treatment of waste material.**

(57)    In a method and apparatus for treating waste material, in particular municipal solid waste (MSW), the MSW is fed through a pre-shredding chamber (12) and into a water jet chamber (14). The jets of water shread the MSW into a slurry which is then passed to a separation chamber (16), from which emerges a stream of clean inert material and a stream of organic biodegradable material. The organic material may be fed to an anaerobic digester (20) to produce biogas which is useable as processing heat.

Fig. 4

### Field of the invention

This invention relates generally to a method of and apparatus for the treatment of waste material, more especially municipal solid waste which is typically collected in black plastics bags.

### Background to the invention

Currently, municipal solid waste generally receives little or no treatment prior to disposal at a landfill site. It is a principal object of this invention to provide a method of and an apparatus for the treatment of municipal solid waste, referred to herein as MSW, more especially to separate inorganic and organic components thereof.

### The invention

According to one aspect of the invention, there is provided a method of treatment of MSW, more especially black bag waste, in which the MSW is fed into a chamber in which pressurised water jets are used to split up and at least complete shredding of the MSW into a mixture comprised by a slurry of fine organic particles mixed with lighter and heavier inert material. The said mixture is then fed through a separation chamber from which emerges a clean inert stream of solid material and an organic biodegradable stream.

According to another aspect of the invention, there is provided apparatus for the treatment of MSW, more especially black bag waste, comprising a chamber incorporating a pressurised water jetting system, means for feeding the MSW into said water jet chamber so that the MSW is split up and shredding thereof completed to produce a mixture comprised by a slurry of fine organic particles mixed wih lighter and heavier inert material, and a separation chamber to which said mixture is fed and from which emerge separate streams of clean inert solid material and of organic biodegradable material.

Optionally, the MSW may first enter a pre-shredding chamber which pulverises the MSW and at least to some extent reduces the size of large items. The pre-shredding chamber may comprise any one or more of a rotating ram, a screw-thread press or a toothed roller shredder. In the case of a rotating ram, the material may be ground against and forced through a very coarse screen, thereafter to enter the water jet chamber which effects cleaning of solid items, cutting of soft materials and completion of shredding.

Pressurised water for forming the jets in the water jet chamber may be generated by direct driven piston pumps (2,000 to 20,000 psi) for low to medium pressure jets or by intensifier pumps (30,000 to 55,000 psi) for high pressure jets. In the latter case, the splitting and cutting action of the jets may be such that a pre-shredding chamber can be dispensed with. Alternatively, pre-shredding may be unnecessary if abrasive water jets (jets in which abrasive particles are entrained) are employed in the water jet chamber.

In any case, the water jetting system in the water jet chamber will preferably comprise a plurality of jets directed at the MSW from the sides and below and may include focussed jets which are directed from the sides normally at the MSW near the top of the water jet chamber, thereby to cut organic material such as paper, fan/shower jets directed from the sides angularly into the main body of material in the chamber, thereby to clean organic material from inorganic material, and mixing jets directed at the MSW from below and designed to generate currents within the chamber such that the MSW is positioned into the optimum orientation for efficient action of the cutting and cleaning jets.

The water jetting system will offer several different combinations of water pressure intensity, jet format and jet positioning in order to enable a required end product to be achieved.

The actual jet nozzles will preferably be of small diameter (0.1 to 0.8 mm), and be made of sapphire.

The separation chamber preferably comprises a float/sink system, so that lighter, mainly plastic, components tend to gather at the top of the chamber and can be removed by a mechanical rake, the organics gather in the central part of the tank and heavier inert material gathers at the bottom, from which this heavy material can be removed through a valve.

Alternatively, a hydrocyclone or a centrifugal system could be used at the separation stage.

From the separation chamber, the organic stream preferably passes, possibly via a water separator, to an anaerobic digester. The digester produces biogas which can be tapped off through a gas holder and a boiler or engine to provide heat and/or electricity, fed back to the digester to assist its digesting action. Heat derived from the extracted biogas may also be applied to the water jet chamber to facilitate the cleaning and cutting actions of the water jets.

The anaerobic digester produces a wet slurry from which water may be extracted at a separator, from which emerges a sludge suitable for use as a soil conditioner and/or a part suitable for disposal thereof at a landfill site.

Extracted water may be filtered and used, in conjunction with fresh water, to provide the water which is pressurised for supply to the water jet chamber.

Reverting to the separator, this also provides a clean stream of inert material which can, if desired, before being disposed of at a landfill site, be sorted and processed for the recovery of re-usable solids such as metals, glass and plastics.

## Description of embodiment

The method and apparatus in accordance with the invention is exemplified with reference to the accompanying drawings, in which:-

Figure 1 is a flowchart showing a basic system;

Figure 2 is a flowchart of a system embodying water recycling;

Figure 3 is a flowchart of a system embodying recycling of extracted heat and generation of electricity; and

Figure 4 shows in principle one embodiment of shredder, water jet chamber, separation chamber, and anaerobic digester for use in the system.

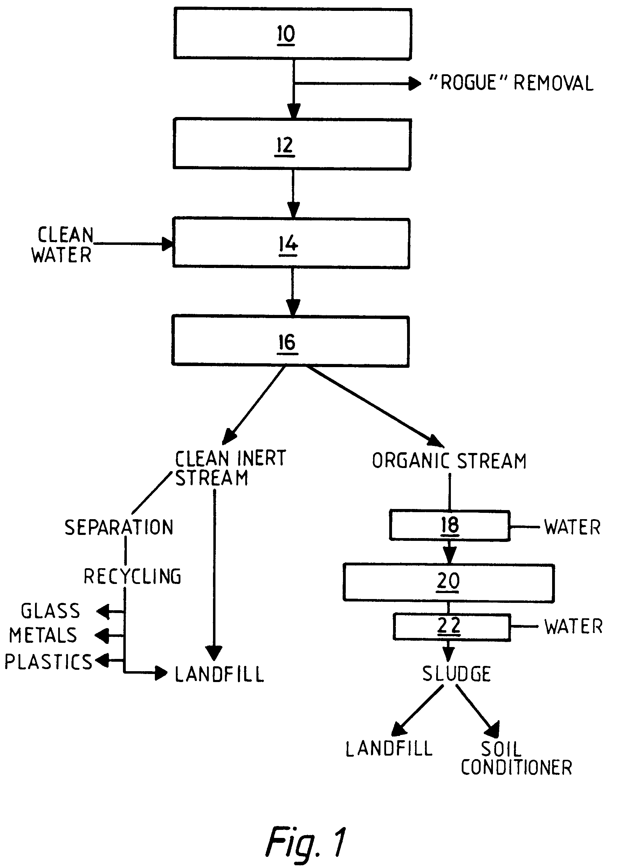

Referring to Figure 1, the basic system as illustrated comprises a source 10 of black bag MSW, for example supplied on a conveyor. After removal of material in the form of large objects such as tree trunks, car engines and the like, MSW first enters a pre-shredding chamber 12, where the waste is crudely shredded, and from the pre-shredding chamber passes to a chamber 14 containing a water jetting system, where shredding is completed and the waste split and cut up, solid items cleaned, and a mixture formed of an organic slurry of fine particles and of inert heavier solid material. The mixture then passes to a separation chamber 16 where the slurry is separated from the heavy inorganic items, which can be removed via a valve at the bottom of this chamber, the organic slurry being taken from near the top.

The clean inorganic waste may now, as indicated, be taken to a landfill site, optionally after sorting and processing to separate out re-usable materials such as metals, glass and plastics.

The organic slurry passes via a separator 18, where water is in part extracted, to an anaerobic digester 20. The digested slurry emergent from the digester then passes to a separator 22, where further water is extracted. The sludge emergent from the separator 22, which is suitable for use as a soil conditioner and for disposal thereof at a landfill site, again as indicated in Figure 1.

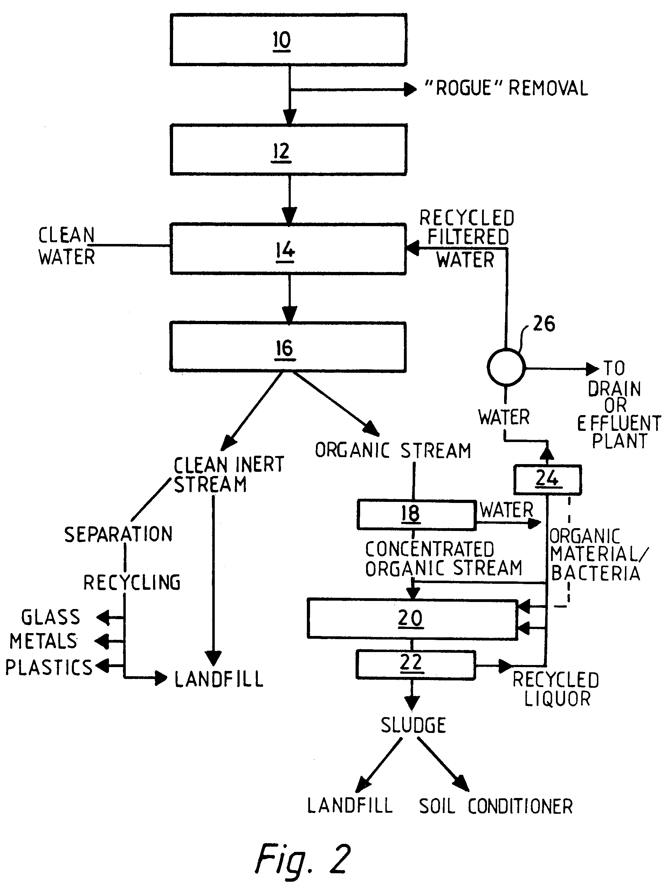

Utilising the same reference numerals, the system is again shown in Figure 2, but with water recycling incorporated. Water extracted at the separators 18 and 22 is fed back through a filter 24 and bleed system 26 for use at the water jet chamber 14.

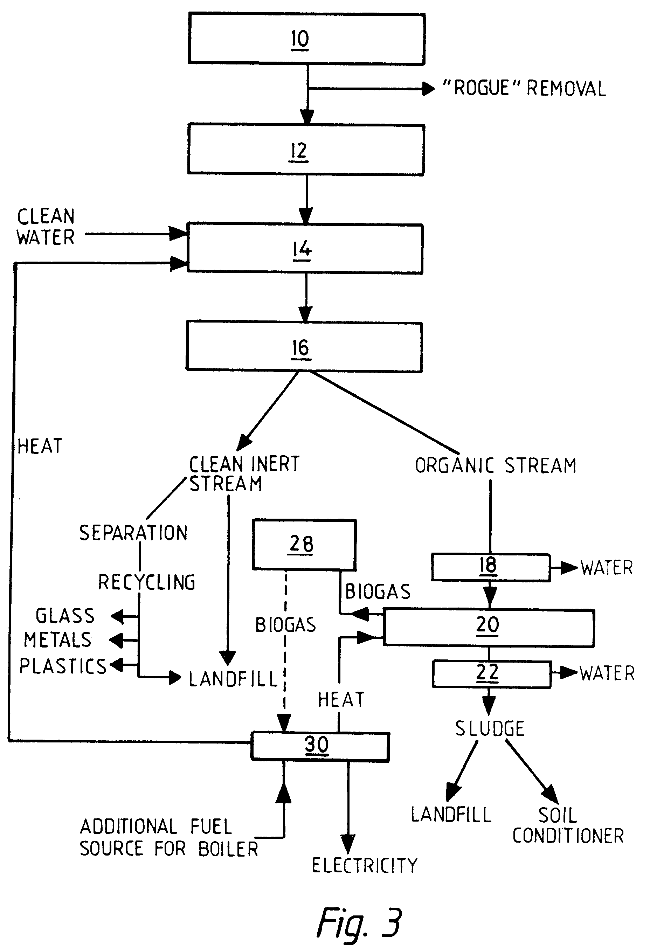

Another modified system is shown in Figure 3, wherein biogas is extracted from the anaerobic digester 20 and utilised, by means of a gas holder 28 and boiler or engine 30, to supply heat to the water jet chamber 14 and the digester.

Clearly, a system is possible in which both water recycling takes place and heat from extracted biogas is utilised within the system.

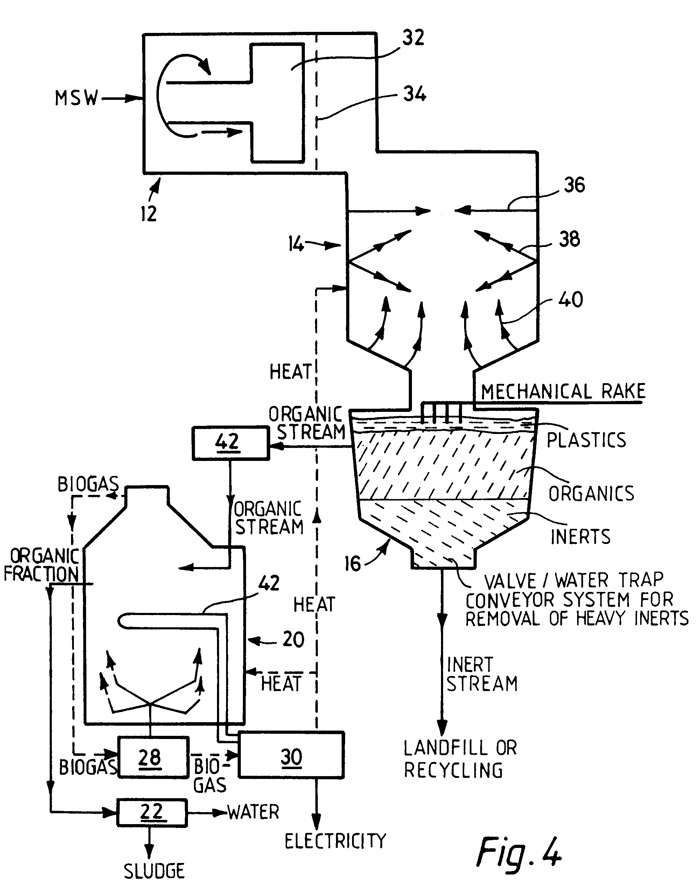

Figure 4 shows the system again, but with an indication of possible embodiments of the pre-shredder 12, the water jet chamber 14, the separation chamber 16 and the anaerobic digester 20.

Examples of systems which might be incorporated in the pre-shredding chamber 12 include:-

1. a hydraulically driven rotary shredder designed for MSW shredding and size reduction;

2. a ram 32 which pulverises material through grate bars 34 into the water jetting chamber, as illustrated in Figure 4;

3. an impact mill;

4. a shredding tool mounted on a rotating rotor;

5. a toothed roller shredder;

6. high pressure water jets;

7. abrasive water jets.

The water jet chamber 14 will incorporate several different types of water jets designed to both cut and slurry organic material and wash organic from inorganic materials. In Figure 4 a water jetting system is indicated which has focussed lateral jets 36 at the top of the chamber, angled fan/shower jets 38 at the sides, and upwardly directed mixing jets 40 at the bottom. Possible types of jet are referred to in more detail later.

High pressure water jets and/or abrasive water jets could be employed within the chamber, for initial shredding of the MSW. In this case an initial shredding chamber would be unnecessary, as shredding could be accomplished in one step.

The three major components of the water jetting system are:

- a source of fluid under pressure, e.g. a pump system;

- nozzles producing jets in the form and quality required; and

- a means of positioning the material requiring cutting and/or cleaning.

Pressurised water is generated either by direct driven piston pumps (2,000 to 20,000 psi) or intensifier pumps (30,000 to 55,000 psi).

Once generated, this pressurised water is expanded in small diameter nozzles with inner diameter 0.1 to 0.8 mm. The nozzles are preferably made of sapphire and serve to transform the potential energy of the water jet into kinetic energy. Thus a high speed water jet is created which accomplishes its cleaning and cutting action through an erosion process.

Low to medium pressures (up to 20,000 psi) are generally employed for cleaning applications whilst higher pressures (20-55,000 psi) are used for cutting applications.

High pressure water jets are capable of cutting relatively soft materials such as plastics, paper, rubber, plywood. In order to cut harder materials such as metals, ceramics etc. abrasive water jets (AWJ) can be employed. In an AWJ system abrasive particles are entrained within the nozzle and accelerated to very high speeds. Typical AWJ speeds are 1,500 to 2,400 feet per second.

Within the water jetting system there are available several different combinations of:-
- pressure intensity;
- jet format; and
- jet positioning;

which can be utilised to give the required end product. This is illustrated in Table 1 below:

Table 1

| Pump type | Pressure | Jet types | Effect on MSW stream |
|---|---|---|---|
| Direct driven piston | 2,000 psi to 20,000 psi | Concentrated straight jets | Cutting/shredding/ pulping organic material garden waste putrescibles/ some paper |
| | | Spread jets − flat sheet fans − solid cones − hollow cones − droplet sprays | Washing, i.e. removal of organics from inert material |
| Inten-sifiers | 30,000 psi to 55,000 psi | Concentrated straight jets | Cutting paper/ plastics/ textiles |
| | | Coherent jets mounted on a rotation head spinning at 500-1000 rpm | |
| | plus abrasives | Concentrated straight jets | Cutting metals, ceramics, plastics, glass |

Thus, the types of jet which may be employed include:
1) Focussed high pressure water jets (up to 40,000 psi) designed to cut through material such as paper, plastics, wood etc.
2) Abrasive water jets designed to cut through hard materials such as metals and ceramics

3) Focussed low to medium pressure water jets (10-20,000 psi) designed to shred/shear and pulp organic materials including paper

4) Low power fan/shower jets designed to clean organic from inorganic material

5) Mixing jets designed to generate currents within the chamber such that material is placed in the correct orientation for optimal operation of the cutting and cleaning etc.

The approximate composition of material typically entering the water jetting chamber is given in Table 2.

Table 2

Average Composition of MSW in the U.K.

| | Percentage | Moisture content |
|---|---|---|
| Paper | 29.2 | 26.6 |
| Putrescibles | 19.0 | 60.3 |
| Wood and garden wastes | 6.0 | − |
| Glass | 8.4 | 1.0 |
| Plastics | 7.0 | 17.2 |
| Metals | 9.0 | 9.2 |
| Textiles | 3.0 | 18.7 |
| Unsorted fines (10mm) | 8.6 | 37.3 |
| Unclassified | 9.8 | 17.8 |
| | | |
| Total dry solids | 67 percent | |
| Moisture | 33 percent | |

Table 3 shows the composition of material leaving the chamber and gives the effect of residence in the water jetting chamber on the various components in the MSW.

Table 3

Organic Slurry - fine particle size

- Putrescibles
- Paper
- Some textiles
- Unsorted fines ( 10mm)
- Garden wastes
- Some wood
- possibly some of the unclassified materials

Inert stream - larger size, heavy fraction and light fraction (plastics)

- Majority of the Wood
- Glass
- Metals
- Plastics
- Possibly some textiles
- Some unclassified material

In the example shown in Figure 4, the separation chamber 16 employs a conventional sink/float system. Here the light plastic fraction gathers at the top of the chamber and can be removed by a mechanical rake system, the organic fraction, which settles in the middle of the chamber, and the heavy inert fraction which gathers at the base of the chamber can be removed through a valve.

In an alternative example the slurry could be passed through a screen which would trap the larger size inert items whilst allowing organic slurry to pass through.

Alternatively a hydrocyclone or centrifugal system could be employed for the separation stage.

Yet again, it would be possible to use a rotating drum with perforated sides in which the organic fraction passes through the holes whilst inert material is retained.

Figure 4 also illustrates one possible configuration of the anaerobic digester 20, which is fed with organic slurry from the separation chamber 16 by means of a feed pump 42. In this embodiment, biogas is extracted and taken to a gas holder 28, and thence to a boiler or engine 30 which provides heat firstly to a heater 42 within the digester and secondly to the water jet chamber.

Various modifications of the above-described and illustrated method and apparatus are possible within the scope of the invention hereinbefore defined.

## Claims

1. A method of treatment of municipal solid waste (MSW), more especially black bag waste, in which the MSW is fed into a chamber in which pressurised water jets are used to split up and at least complete shredding of the MSW into a mixture comprised by a slurry of fine organic particles mixed with lighter and heavier inert material.

2. A method according to claim 1 further comprising the step of feeding the mixture through a separation chamber for separating said mixture into a clean inert stream of solid material and an organic biodegradable stream.

3. Apparatus for the treatment of municipal solid waste (MSW), more especially black bag waste, comprising a water jet chamber incorporating a pressurised water jetting system, means for feeding the MSW into said water jet chamber so that the MSW is split up and shredding thereof completed to produce a mixture comprised by a slurry of fine organic particles mixed with lighter and heavier inert material, and a separation chamber to which said mixture is fed and from which emerge separate streams of clean inert solid material and of organic biodegradable material.

4. Apparatus according to claim 3 further comprising a pre-shredding chamber, upstream of said water jet chamber, which pulverises the MSW and at least to some extent reduces the size of large items.

5. Apparatus according to claim 4 in which the pre-shredding chamber comprises at least one of a rotating ram, a screw-thread press and a toothed roller shredder.

6. Apparatus according to the claim 5 comprising a rotating ram, the material being ground against and forced through a very coarse screen, thereafter to enter the water jet chamber which effects cleaning of solid items, cutting of soft materials and completion of shredding.

7. Apparatus according to any one of claims 3 to 6 in which pressurised water for forming the jets in the water jet chamber is generated by direct driven piston pumps (2,000 to 20,000 psi) for low to medium pressure jets, or by intensifier pumps (30,000 to 55,000 psi) for high pressure jets.

8. Apparatus acording to any one of claims 3 to 7 in which abrasive particles are entrained in the water jets in the water jet chamber.

9. Apparatus according to any one of claims 3 to 8 in which the water jetting system in the water jet chamber comprises a plurality of jets directed at the MSW from the sides and below, and include focussed jets which are directed from the sides normally at the MSW near the top of the water jet chamber, thereby to cut organic material such as paper, fan/shower jets directed from the sides angularly into the main body of material in the chamber, thereby to clean organic material from inorganic material, and mixing jets directed at the MSW from below and designed to generate currents within the chamber such that the MSW is positioned into the optimum orientation for efficient action of the cutting and cleaning jets.

10. Apparatus according to any one of claims 3 to 9 in which the water jets are formed by nozzles of 0.1 to 0.8mm diameter, and made of sapphire.

11. Apparatus according to any one of claims 3 to 10 which the separation chamber comprises a float/sink system, so that lighter, mainly plastic, components tend to gather at the top of the separation chamber, a mechanical rake for removing said components, the organic material gathering in the central part of the chamber and heavier inert material gathering at the bottom, from which this heavy material can be removed through a valve.

12. Apparatus according to any one of claims 3 to 10 in which a hydrocyclone or a centrifugal system is used at the separation stage.

13. Apparatus according to any one of claims 3 to 12 in which said organic biodegradable stream passes, optionally via a water separator, to an anaerobic digester.

*Fig. 1*

```
                    ┌─────────────────────┐
                    │         10          │
                    └─────────┬───────────┘
                              │──────────────────────► "ROGUE" REMOVAL
                              ▼
                    ┌─────────────────────┐
                    │         12          │
                    └─────────┬───────────┘
                              │
                              ▼                   RECYCLED
CLEAN               ┌─────────────────────┐       FILTERED
WATER ─────────────►│         14          │◄──────  WATER
                    └─────────┬───────────┘
                              │
                              ▼
                    ┌─────────────────────┐               26
                    │         16          │              ◯──► TO DRAIN
                    └──────┬───────┬──────┘                      OR
```

CLEAN INERT STREAM          ORGANIC STREAM        WATER       EFFLUENT PLANT

SEPARATION

RECYCLING

GLASS ◄

METALS ◄

PLASTICS ◄──► LANDFILL

CONCENTRATED ORGANIC STREAM

ORGANIC MATERIAL/BACTERIA

RECYCLED LIQUOR

| 18 | WATER
| 24 |
| 20 |
| 22 |

SLUDGE

LANDFILL     SOIL CONDITIONER

## Fig. 2

Fig. 3

MSW →

32

34

12

14

36

38

40

HEAT

ORGANIC
STREAM

42

MECHANICAL RAKE

PLASTICS

ORGANICS

INERTS

BIOGAS

ORGANIC
STREAM

16

VALVE / WATER TRAP
CONVEYOR SYSTEM FOR
REMOVAL OF HEAVY INERTS

ORGANIC
FRACTION

42

HEAT

20

HEAT

INERT
STREAM

LANDFILL OR
RECYCLING

BIOGAS

28

BIO
GAS

30

22 → WATER

ELECTRICITY

Fig. 4

SLUDGE